# EUROPEAN PATENT APPLICATION

(11) **EP 2 085 798 A1**
(43) Date of publication of application: **05.08.2009**
(21) Application number: 06841678.3
(22) Date of filing: 02.11.2006
(51) Int. Cl.: G02B 5/23, F21V 9/00, A61F 9/00

(54) **LIGHTING DEVICE WITH PROPHYLACTIC AND THERAPEUTIC FILTER FOR HEALTHY EYES, PSEUDOAPHAKIC EYES AND/OR EYES SUFFERING NEURODEGENERATION**

(30) Priority: 16.10.2006 ES 200602621
(71) Applicant: Universidad Complutense De Madrid, 28040 Madrid (ES)
(72) Inventor: SANCHEZ RAMOS, Celia, 28040 Madrid (ES)
(74) Representative: Temino Ceniceros, Ignacio
(86) International application number: PCT/ES2006/000606
(87) International publication number: WO 2008/046933

(57) **Abstract**

The invention relates to a device for the therapeutic and prophylactic treatment of healthy eyes, pseudo-aphakic eyes and/or eyes with macular and retinal degeneration, **characterised in that** it is produced by applying a filter with yellow pigmentation to an ordinary lighting device in order to protect the eyes from short wavelengths in the visible spectrum (less than 500 nm). The invention removes the difficulties and risks associated with existing techniques used to provide protection for catacract-operated eyes and to protect and improve healthy eyes and eyes suffering neurodegenerative processes, simply by applying a protective filter to any lighting device. The invention combines an ordinary lighting device with a yellow pigmentation filter which absorbs short wavelengths of less than 500 nm.

## Description

### OBJECT OF THE INVENTION

The invention is part of the ophthalmology sector, within optic applications that are therapeutic and prophylactic in nature.

The purpose of this invention is to develop a device, which protects healthy eyes, pseudo-aphakic (operated of cataracts) and/or with macular and retina degeneration, that accomplishes this by applying a filter with yellow pigmentation over an illumination device with the objective of protecting them from visible spectrum short wavelengths (of less than 500nm).

### STATE OF THE ART

Perceiving vision is the result of the response to visible radiation (380-760nm). In the environment, solar radiation supposes the main risk to vision. The sun emits UV and IR radiations, which are almost entirely absorbed by the atmosphere. The solar radiation that is transmitted through the atmosphere when it reaches the surface of the earth, consists of UV-B rays (230-300nm), UV or UV-A rays (300-380 nm), visible light (380-760 nm) and IR rays (760-1400 nm). Human eyes that are in perfect health conditions freely transmit the IR rays and most of the visible spectrum to the retina but the cornea and the crystalline prevents the visible spectrum reactive mss waves (the UV-B rays and the portion of blue light from the visible spectrum) from reaching the retina.

On the other hand, the human crystalline changes its transmission characteristics as it ages, intensifying its yellow color and incrementing its capacity for filtering UV and blue light rays. For this reason, the violet rays (<400nm) is not transmitted in people older than 65 and the transmission of blue light (400 - 500 nm) is significantly reduced.

On the other hand, the retina auto-protects itself from the short wavelengths in two ways: with a heterogeneous distribution of the photo-receptors in such a way that photo-receptors, which are sensitive to blue light, do not exist in the macular depression and by the action of existing yellow pigments in the same area that also perform a protective action.

These natural protections of the human eye against the shortest wavelengths -the crystalline and those of the retina- can find themselves seriously affected by certain pathologies and/or surgical interventions:
- Cataracts, which can only be treated surgically requires the extraction of the crystalline.
- The appearance of a pathological aging process occurs frequently and produces a degradation of the retina structures producing age related macular degeneration (AMD).

Historically, we must consider the convergence in the same demographic group (persons older than 65 years) of these two pathologies: the cataract and the AMD. Cataracts are the main cause of loss of vision and the AMD of blindness in this same population segment. Additionally, we must consider the presumable increment of both pathologies due, among other things, to the increase of life expectancy and therefore these provoke a great interest in investigators and their application in the optic industry.

Therefore, as explained in the scientific bibliography, several epidemiological studies have been evaluated by the cataract surgery association and the age related macular degeneration (AMD). The works by Klein (Klein R, Klein BE, Wong TY, Tomany SC, Cruickshanks KJ. The association of cataract and cataract surgery with the long-term incident of age-related maculopathy. Arch Ophthalmol 120:1551-1558.2002) and Freeman (Freeman E, Munoz B, West SK, Tielsch JM, Schein OD. Is there an association between cataract surgery and age-related macular degeneration? Am J Ophthalmolm 135(6): 849-856.2003) assure the existence of a higher risk of developing the AMD symptoms in people who have had cataract surgery. However, the prior investigations by Wang (Wang JJ, Mitchell P, Cumming RG, Lim R. Cataract and age related maculopathy: the Blue Mountains Eye Study. Ophthalmic Epidemiol 6: 317-326.1999) and McCarty (McCarty CA, Mukesh BN, Fu CL, Mitchell P, Wang JJ, Taylor HR. Risks factors for age-related maculopathy: the Visual Impairment Project. Arch Ophthalmol 119:1455-1462.2001) reject this hypothesis, possibly due to a less developed level of applied technology for diagnostic measurements. It is only recently that the implementation of techniques like optic coherent Tomography that in a rigorous way allows to immediately and non invasively perform follow up of the neuro-degenerative retina process evolution. This is an important fact for knowing the determining effect of the natural pigments that absorb harmful radiations.

On the other hand, some techniques have been developed to protect cataract operated eyes from short wavelengths:
- Several types of filters exist in the market, which are provided with yellow pigmentation, but there still isn't an optimum procedure and/or device to apply these filters to the human eyes as a therapeutic and preventive measure to substitute and/or improve the natural protection.
- Since the middle of the 90's, intraocular lenses provided with a yellow filter have been implanted on cataract operated eyes. This alternative involves a surgical procedure with all its obvious risks and difficulties. There also exists a large number of people operated from cataracts to which a transparent intraocular lens has been implanted to substitute the crystalline that does not have the necessary yellow pigmentation protection. In these cases, it is necessary to complement the artificial crystalline, which is exempt of yellow pigmentation, with the insertion of a yellow pigmentation support system.

Also, several patents related to this technique have been developed, which present significant differences when compared to this invention.
- Absorption filter for color display devices (patent number US5121030), which through the application of tints, improves visibility during conditions of high illumination intensity levels.
- Color enhancement filter and instructions for use for improving vision in the human eye (patent number US6158865), which includes a filter to improve vision in all light environments, including extreme ambient lighting and low levels of illumination and incorporate a ring adapter for the filter.
- Specific Optic Filters for certain activities and Optic accessories which use these filters (patent number US6893127), which improve the visualization of objects for example, in sports.
- Optic filter and device, heat rays absorbent filter, fiber optic and glasses provided with this optic filter (patent number WO9927397), which is composed of a synthetic resin capable of protecting from infrared light rays.
- Method for the design of color filters that improve or modify the color vision in the human eye and filtering methods for color designed by the method (patent number US2004075810).
- System and method for applying correction factors related to environmental conditions (patent number US2006195278), based on a color meter programmed via software and/or hardware to compensate for environmental factors.
- Protection solution for eye treatment (patent number WO2005025575), specifically made of a viscous-elastic liquid or flush that contains substances that, at least in part, filter the luminous radiation from specific frequencies.
- Human eye protection and correction device that includes assembling of filters to protect against electromagnetic radiation and/or correcting vision abnormalities, like myopia and loss of color vision (patent number DE10259261).
- Optic vision device that includes an apparatus for partially reducing illumination intensity (patent number US2002113941), for example a surgical microscope that includes a spectral filter adapted to reduce, without eliminating the intensity of the light emitted by a source in a specific region of the object (which can be the human eye).
- Illumination intensity detection and control system for ocular and projection microscopic lamps (patent number US6299310, based on patent US4715704) that on one hand allows working with a high level of illumination on the eye that is subject to exam and on the other hand prevents damaging the eye.
- Diffusion Platelet in combination with a microscopic lamp (patent number DE8808871) that controls the illumination emitted by the lamp.
- LED system (light emitting diode) for ocular exams (patent number IT1147092), than may include filters.
- Solid photometer - device for detecting ocular and optic nerve deficiencies (patent number JP5130976), that includes the utilization of neutral intensity filters.

These patents are different from this invention fundamentally in their objectives and usefulness since none of them have the purpose of protecting healthy human eyes with a neurodegenerative process or those that have been operated of cataracts against short wavelengths. Also, most of them make no reference to applying a filter over a common lamp, instead, they materialize on other types of formats (light specific devices, lenses, solutions...).

### DESCRIPTION OF THE INVENTION

The objective of this invention in the case of pseudo-aphakic subjects, is to functionally compensate for the extraction of pigment protectors (extracted during the surgical process) and in the case of healthy eyes or with degenerative processes is to enhance the prophylactic effect of blue and violet light rays by using a filter applied to the illumination device/s. As mentioned before, these two processes frequently coincide in the same population group, the elderly.

For this, the invention consists of an illumination device that protects the pathological or healthy eyes from neuro-degenerative processes due to the absorption of harmful light rays, which is obtained as a result of applying a filter with yellow pigmentation to absorb the short wavelengths between 350 and 500 nm applied to a common illumination device.

Therefore, the device incorporates a combination of three elements:
- A common illumination device such as incandescent, fluorescent or halogen lamps.
- A mount or support to apply the filter to the illumination device lamp.
- Application of a filter with yellow pigmentation, available on the market and compatible with the illumination device to absorb the short wavelengths between 350 and 500 nm, on all the illumination device's emission area.

### EMBODIMENT OF THE INVENTION

Several methods exist for applying this invention depending on the type of illumination device. Additionally, the application method for this invention is illustrated in the following example, which is not limiting in scope since there are alternative means and combinations for manufacturing this device.

Invention manufacturing example:
- Prepare a filter that is yellow in color from the ones available on the market, for example, shaped as a screen or tint and compatible with the illumination device.
- Prepare support material from those that are available on the market for applying the filter to the illumination lamp device in accordance with the manufacturer instructions or for example, based on patents EO1830250 (filter support junction for illumination devices) or ES1046793 (filter support structure applied over illumination lamps).
- The yellow filter is installed over the support material in such a way that it covers the entire illumination device's light emission area.

In short, the combination of an illumination device and a yellow filter will allow patients that have been operated from cataracts with an intraocular transparent lens to correct the fact that the operated eye is not protected, and eyes with neuro-degenerative processes improve and increase in this way their natural protection, and for healthy eyes, prevent neuro-degenerative processes from occurring. In this way, the problems associated with alternative techniques that exist on the market and require surgical interventions with intraocular lenses are avoided.

## Claims

1. Therapeutic and prophylactic device for the protection of pseudo-aphakic eyes **characterized** for being the result of applying a filter with yellow pigmentation that absorbs short wavelengths between 350 and 500 nm over one or several illumination devices.

2. Therapeutic and prophylactic device for pseudo-aphakic eyes as per claim 1 that corresponds to a filter with yellow pigmentation that is suitable to be used on the illumination device.

3. Therapeutic and prophylactic device for pseudo-aphakic eyes as per claims 1 and 2 that includes a common illumination device.

4. Therapeutic and prophylactic device for healthy eyes or those with neuro-degenerative retina processes **characterized** for being the result of applying a filter with yellow pigmentation that absorbs short wavelengths between 350 and 500 nm over one or several illumination devices.

5. Therapeutic and prophylactic device for healthy eyes or those with neuro-degenerative retina processes as per claim 4 that corresponds to a filter with yellow pigmentation that is suitable to be used on the illumination device.

6. Therapeutic and prophylactic device for healthy eyes or those with neuro-degenerative retina processes as per claims 4 and 5 that includes a common illumination device.
